# EUROPEAN PATENT SPECIFICATION

(11) **EP 3 143 991 B1**
(45) Date of publication and mention of the grant of the patent: **08.04.2020**
(21) Application number: 16189181.7
(22) Date of filing: 16.09.2016
(51) Int. Cl.: A61K 9/16, A61K 9/48, A61K 31/137

(54) **FINGOLIMOD CAPSULE COMPOSITION**
FINGOLIMODKAPSELZUSAMMENSETZUNG
COMPOSITION POUR CAPSULE DE FINGOLIMOD

(30) Priority: 18.09.2015 TR 201511720
(43) Date of publication of application: 22.03.2017
(73) Proprietor: Sanovel Ilac Sanayi ve Ticaret A.S., 34460 Istanbul (TR)
(72) Inventor: TÜRKYILMAZ, Ali, 34460 Istanbul (TR); ZENGINER, Sibel, 34460 Istanbul (TR); DEDEOGLU, Yavuz, 34460 Istanbul (TR); YAG, Göksu, 34460 Istanbul (TR); ÖZTÜRK, Erkin, 34460 Istanbul (TR)
(74) Representative: Sevinç, Erkan

(56) References cited:
- WO-A1-2013/019872
- WO-A1-2015/015254
- IN-A- 3428M UM2 013
- US-A1- 2015 141 520
- Anonymous: "Avicel for Solid Dose Forms", FMC Health and Nutrition , 2016, pages 1-2, XP002764813, Retrieved from the Internet: URL:http://www.fmcbiopolymer.com/Pharmaceu tical/Products/Avicelforsoliddoseforms.asp x [retrieved on 2016-11-23]

## Description

### Field of Invention

The present invention relates to a pharmaceutical capsule composition comprising fingolimod or its pharmaceutically acceptable salts thereof cellulose derivatives excipient, a binder and at least one pharmaceutically acceptable excipient, wherein said binder is PEG6000.

### Background of Invention

Multiple sclerosis (MS) is the most common autoimmune disorder affecting the central nervous system. Multiple sclerosis, also known as disseminated sclerosis or encephalomyelitis disseminata, is a demyelinating disease in which the insulating covers of nerve cells in the brain and spinal cord are damaged. This damage disrupts the ability of parts of the nervous system to communicate, resulting in a wide range of signs and symptoms, including physical, mental and sometimes psychiatric problems. MS takes several forms, with new symptoms either occurring in isolated attacks (relapsing forms) or building up over time (progressive forms). Between attacks, symptoms may disappear completely; however, permanent neurological problems often occur, especially as the disease advances.

Fingolimod is an immunomodulating drug, mostly used for treating multiple sclerosis (MS). Fingolimod is asphingosine-1-phosphate receptormodulator, which sequesters lymphocytes in lymph nodes, preventing them from contributing to an autoimmune reaction. Fingolimod is used in the treatment of the relapsing form of multiple sclerosis. May also be used in chronic inflammatory demyelinating polyneuropathy.

Fingolimod is marketed by Novartis under the brand name Gilenya® for the treatment of multiple sclerosis Gilenya® is presented as immediate-release hard gelatin capsules containing 0.56 mg of fingolimod hydrochloride as the active substance corresponding to 0.5 mg of fingolimod base.

Fingolimod is 2-amino-2-[2-(4-octylphenyl)ethyl]propane-1,3-diol and shown as Formula I.

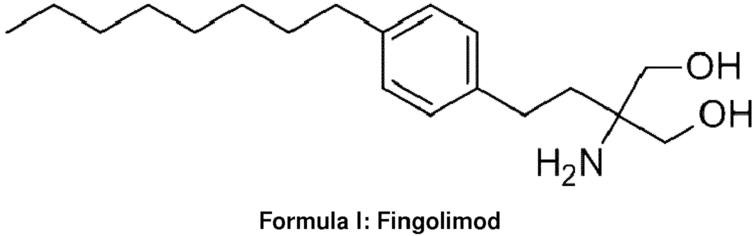

In prior art, fingolimod derivatives first disclosed in US5604229. The use of fingolimod derivatives as immunodepressant and a preventive or remedy for autoimmune diseases is disclosed in EP0627406 (B1). The use of fingolimod derivatives in the prevention or treatment of chronic rejection in a recipient of organ or tissue allo- or xenotransplant is disclosed in EP0941082 (B1).

WO201319872 addresses the problem of stability by mixing fingolimod with dry surfactant and then formulating the resulting pre-mix.

1844/CHE/2011 discloses a solid oral composition comprising fingolimod, pregelatinized starch and stearic acid, but dissolution profile of this composition is slower when compared with Gilenya® product. Moreover, patent application does not provide any stability study to show compatibility of excipients with Fingolimod.

It has been discovered that fingolimod possess properties that can cause several processing problems like content uniformity as fingolimod particles have strong tendency to stick surfaces and each other.

Despite of various prior arts availability to provide solution of one or the other issues associated with fingolimod hydrochloride, still there exists a need for selecting excipient/s for formulating fingolimod, which provides a stable product and provides desired therapeutic effect.

Present invention overcomes all the above mentioned problems by providing a stable solid oral capsule composition having desired content uniformity and dissolution profile. It can be dispensed as an oral dosage form to overcome the several processing problem associated with the fingolimod composition.

US 2015/141520 A1 relates to pharmaceutical compositions comprising a SIP receptor modulator; in particular US 2015/141520 A1 discloses fingolimod compositions comprising at least one excipient which is not a sugar alcohol.

WO 2015/015254 A1 relates to a pharmaceutical composition comprising fingolimod, a pharmaceutically acceptable salt thereof or a phosphate derivative as an active ingredient and its preparation.

IN 3428 MUM 2013 A1 relates to solid oral dosage forms comprising effective amounts of fingolimod or salts thereof.

### Description of the invention

This invention is about a pharmaceutical capsule composition comprising fingolimod or its pharmaceutically acceptable salts thereof, cellulose derivatives excipient, a binder and at least one pharmaceutically acceptable excipient, wherein said binder is PEG6000.

In a preferred embodiment, pharmaceutically acceptable salt of fingolimod is fingolimod hydrochloride.
In the marketed Fingolimod product (Gilenya®) the capsule comprises mannitol as diluent, prepared by direct blending method and capsule additionally comprises small amount of magnesium stearate as a lubricant. Fingolimod HCI was used in a micronized form to ensure content uniformity of the active substance. It is disclosed in literature that fingolimod reacts with several excipients during compatibility studies and thus mannitol was used as a diluent in Gilenya® composition due to the optimum degradation profile. Since, dose of the approved product is very low, and filler or diluent makes most of the part of the composition it is essential to select excipient which is highly compatible with API. (European public assessment report of Gilenya®)
Both in the marketed Fingolimod product and in this present invention, the hard gelatin capsules containing 0.56 mg of fingolimod hydrochloride as the active substance corresponding to 0.5 mg of fingolimod base. The proportion of the active agent in the total weight of the composition in the case of compositions for oral administration is typically in the range of only a few per cent by weight, such as 0.25 to 4 % by weight. This small proportion of active agent can lead to considerable problems during the manufacture of the composition with regard to the uniformity of the content of active agent in the individual composition units. The compatibility studies showed that the active substance interacts with several excipients. It reflects that content uniformity play important role in the dissolution of the drug.
According to this embodiment, the amount of fingolimod hydrochloride is present in an amount of 0.25 to 4 % by weight of total composition; preferably it is 0.5 to 2.5% by weight of total composition.
The present invention is based on the findings that pharmaceutical compositions and dosage forms comprising fingolimod hydrochloride having improved content uniformity and/or flowability and/or dissolution by using cellulose and/or its derivatives, chosen based on compatibility studies and experience from development of similar compositions.

In this present invention, microcrystalline cellulose PH 101 is used in in this present capsule composition to improve flow, facilitate plug formation and aid capsule disintegration.

According to a preferred embodiment, the pharmaceutical capsule composition comprises cellulose derivatives excipient is microcrystalline cellulose PH 101.

In one embodiment, the amount of microcrystalline cellulose PH 101 is present in an amount of 35.0 to 75.0 % by weight of total composition; preferably it is 45.0 to 65.0% by weight of total composition.

An object of the present invention is to obtain adequate content uniformity and desired particle size distribution of composition comprising fingolimod hydrochloride and microcrystalline cellulose PH 101 and improved processes. In this invention, the composition has homogeneity and high flowability properties during the process.

As used here in, "particle size distribution" is defined by the cumulative volume size distrubition as tested by a conventionally accepted method which is the laser diffraction method determined by the equipment of Malvern Mastersizer 2000. "Median particle size" is defined by "d50", the diameter where fifty percent of the distribution has a smaller particle size and "d90" means that the diameter where ninety percent of the distribution has a smaller particle size. "Desired particle size distribution" is defined by the ratio between the median particle size (d50) and the particle size at (d90).

In one embodiment, the pharmaceutical capsule composition comprising microcrystalline cellulose PH 101 having a particle size (d₅₀) between 50.0 µm and 95.0 µm, and preferably between 60.0 µm and 85.0 µm.

In another embodiment, the pharmaceutical capsule composition comprising microcrystalline cellulose PH 101 having a particle size (d₉₀) between 125.0 µm and 165.0 µm, and preferably between 135.0 µm and 155.0 µm.

According to the special particle size of microcrystalline cellulose PH 101 in the composition, the invention's advantages are resonable preparing process, high flowability, uniform dispersion and desired dissolution profile. It has been found that this specific particle size improves content uniformity and flowability. In this present invention, the composition can be easily handled, mixed with the excipients having particular particle size and can be processed into pharmaceutical product without the known and expected problems such as segregation or de-mix during the process steps.
According to one embodiment, the pharmaceutical capsule composition comprises at least one pharmaceutically acceptable excipient is selected from diluent, binder, lubricant or mixtures thereof.
Suitable diluents may comprise but not limited to microcrystalline cellulose, microcrystalline cellulose PH 101, calcium hydrogen phosphate dihydrate, lactose spray dried, mannitol, spray-dried mannitol, lactose, starch, maize starch, dextrose, sucrose, fructose, maltose, sorbitol, xylitol, inositol, kaolin, inorganic salts, calcium salts, polysaccharides, calcium phosphate, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to another preferred embodiment, the pharmaceutical capsule composition comprises diluent is calcium hydrogen phosphate dihydrate.
In one embodiment, the amount of calcium hydrogen phosphate dihydrate is present in an amount of 20.0 to 60.0 % by weight of total composition; preferably it is 30.0 to 50.0% by weight of total composition.

In this present invention, polyethylene glycol 6000 P is selected as a suitable binder. Polyethylene glycol 6000 P is a polethylene glycol with a mean molecular weight of 6000. It is a solid in powder form. It has very low water content and excellent solubility in water. In this present invention, polyethylene glycol 6000 P is used in the wet granulation process, the risk of over-granulation is reduced, screen blockage is avoided and uniform, rapid drying is achieved.

In one embodiment, the amount of polyethylene glycol 6000 P is present in an amount of 0.5 to 5.0 % by weight of total composition; preferably it is 1.0 to 4.0% by weight of total composition.

Suitable lubricants may comprise but not limited to sodium stearyl fumarate, magnesium stearate, calcium stearate, zinc stearate, waxes, boric acid, hydrogenated vegetable oil, sodium chlorate, magnesium lauryl sulfate, sodium oleate, sodium acetate, sodium benzoate, stearic acid, fatty acid, fumaric acid, glyseryl palmito sulphate, sodium lauryl sulphate or mixtures thereof.

In this present invention, sodium stearyl fumarate is selected as a suitable lubricant. Due to hydrophilic property of sodium stearyl fumarate, composition does not have the disadvantages of magnesium stearate in respect of flowability and dissolution. In one aspect, the present invention relates to fingolimod hydrochloride capsule composition which does not comprise magnesium stearate. Magnesium stearate is a metal derivative lubricant. It has anti-adhesive and flow enhancement properties by ensuring uniformity. Besides this advantageous, lubricant forms a hydrophobic film around the active agent and retards the dissolution by retarding wetting of active agent. In this invention, sodium stearyl fumarate helps to achieve desired dissolution profile.

In one embodiment, the amount of sodium stearyl fumarate is present in an amount of 0.5 to 5.0 % by weight of total composition; preferably it is 1.0 to 4.0% by weight of total composition.

In one embodiment, the pharmaceutical capsule composition consisting of;
**a.** fingolimod hydrochloride is present in an amount of 0.25 to 4 % by weight of total composition; preferably it is 0.5 to 2.5% by weight of total composition
**b.** microcrystalline cellulose PH 101 is present in an amount of 35.0 to 75.0 % by weight of total composition; preferably it is 45.0 to 65.0% by weight of total composition
**c.** calcium hydrogen phosphate dihydrate is present in an amount of 20.0 to 60.0 % by weight of total composition; preferably it is 30.0 to 50.0% by weight of total composition
**d.** polyethylene glycol 6000 P is present in an amount of 0.5 to 5.0 % by weight of total composition; preferably it is 1.0 to 4.0% by weight of total composition
**e.** sodium stearyl fumarate is present in an amount of 0.5 to 5.0 % by weight of total composition; preferably it is 1.0 to 4.0% by weight of total composition

In a preferred embodiment, the pharmaceutical capsule composition comprising;
**a.** fingolimod hydrochloride
**b.** microcrystalline cellulose PH 101 having a particle size (d₅₀) between 50.0 µm and 95.0 µm, and preferably between 60.0 µm and 85.0 µm.
**c.** calcium hydrogen phosphate dihydrate
**d.** polyethylene glycol 6000 P
**e.** sodium stearyl fumarate

Another object of the present invention is to provide a pharmaceutical capsule composition comprising fingolimod or its pharmaceutically acceptable salt thereof for the treatment of multiple sclerosis, preferably relapsing-remitting multiple sclerosis.

### Example : Fingolimod hydrochloride capsule composition

Microcrystalline cellulose PH 101: d₅₀= 50.0 µm - 95.0 µm ; d₉₀= 125.0 µm - 165.0 µm

| **Ingredients** | **Amount (mg)** |
|---|---|
| Fingolimod hydrochloride | 0.56 |
| Microcrystalline cellulose PH 101 | 27.44 |
| Calcium hydrogen phosphate dihydrate | 20.00 |
| Polyethylene glycol 6000 P | 1.00 |
| Sodium stearyl fumarate | 1.00 |

**Manufacturing Process:** Fingolimod hydrochloride, microcrystalline cellulose PH 101, calcium hydrogen phosphate dihydrate and polyethylene glycol 6000 P is blended progressively. The mixture is granulated with ethanol: water (1:1) solution. The granules were dried at 35 °C and sieved. Sodium stearyl fumarate is added to the final mixture. Then the powder is filled to the hard gelatin capsules.

## Claims

1. A pharmaceutical capsule composition comprising fingolimod or its pharmaceutically acceptable salts thereof, cellulose derivatives excipient, a binder and at least one pharmaceutically acceptable excipient; wherein said binder is polyethylene glycol 6000 P.

2. The pharmaceutical capsule composition according to claim 1, wherein pharmaceutically acceptable salt of fingolimod is fingolimod hydrochloride.

3. The pharmaceutical capsule composition according to claim 1, wherein cellulose derivatives excipient is microcrystalline cellulose PH 101.

4. The pharmaceutical capsule composition according to claim 3, wherein microcrystalline cellulose PH 101 is present in an amount of 35.0 to 75.0 % by weight of total composition.

5. The pharmaceutical capsule composition according to claim 3, wherein microcrystalline cellulose PH 101 having a particle size (d₅₀) between 50.0 µm and 95.0 µm.

6. The pharmaceutical capsule composition according to claim 1, wherein at least one pharmaceutically acceptable excipient is selected from diluent, lubricant or mixtures thereof.

7. The pharmaceutical capsule composition according to claim 6, wherein diluent is calcium hydrogen phosphate dihydrate.

8. The pharmaceutical capsule composition according to claim 6, wherein lubricant is sodium stearyl fumarate.

9. The pharmaceutical capsule composition according to any of the preceding claims comprising;
**a.** fingolimod hydrochloride
**b.** microcrystalline cellulose PH 101 having a particle size (d50) between 50.0 µm and 95.0 µm
**c.** calcium hydrogen phosphate dihydrate
**d.** polyethylene glycol 6000 P
**e.** sodium stearyl fumarate

## Patentansprüche

1. Pharmazeutische Kapselzusammensetzung mit Fingolimod oder ihren pharmazeutisch akzeptablen Salzen hierzu, Cellulosederivate-Hilfsstoff, einem Binder und mindestens einem pharmazeutisch akzeptablen Hilfsstoff,
wobei der Binder Polyethylenglycol 6000 P ist.

2. Pharmazeutische Kapselzusammensetzung nach Anspruch 1,
wobei ein pharmazeutisch akzeptables Salz von Fingolimod-Hydrochlorid ist.

3. Pharmazeutische Kapselzusammensetzung nach Anspruch 1,
wobei ein Cellulosederivate-Hilfsstoff eine mikrokristalline Cellulose PH 101 ist.

4. Pharmazeutische Kapselzusammensetzung nach Anspruch 3,
wobei eine mikrokristalline Cellulose PH 101 in einer Menge von 35.0 bis 75.0% an dem Gesamtgewicht vorhanden ist.

5. Pharmazeutische Kapselzusammensetzung nach Anspruch 3,
wobei eine mikrokristalline Cellulose PH 101 eine Partikelgröße (d₅₀) zwischen 50.0 µm und 95.0 µm besitzt.

6. Pharmazeutische Kapselzusammensetzung nach Anspruch 1,
wobei zumindest ein pharmazeutisch akzeptabler Hilfsstoff ausgewählt ist aus Verdünnungsmittel, Gleitmittel oder Mischungen hiervon.

7. Pharmazeutische Kapselzusammensetzung nach Anspruch 6,
wobei das Verdünnungsmittel Kalziumhydrogen-Phosphatdihydrat ist.

8. Pharmazeutische Kapselzusammensetzung nach Anspruch 6,
wobei das Gleitmittel Natrium-Stearylfumarat ist.

9. Pharmazeutische Kapselzusammensetzung nach einem der vorhergehenden Ansprüche, mit:
a) Fingolimod-Hydrochlorid,
b) mikrokristalline Cellulose PH 101 mit einer Partikelgröße (d50) zwischen 50.0 µm und 95.0 µm,
c) Kalziumhydrogen-Phosphatdihydrat,
d) Polyethylenglykol 6000 P,
e) Natrium-Stearylfumarat.

## Revendications

1. Composition pour capsule pharmaceutique comprenant du fingolimod ou les sels pharmaceutiquement acceptables de celui-ci, un excipient dérivé de cellulose, un liant et au moins un excipient pharmaceutiquement acceptable, ledit liant étant le polyéthylène glycol 6000 P.

2. Composition pour capsule pharmaceutique selon la revendication 1, dans laquelle le sel pharmaceutiquement acceptable de fingolimod est le chlorhydrate de fingolimod.

3. Composition pour capsule pharmaceutique selon la revendication 1, dans laquelle l'excipient dérivé de cellulose est la cellulose microcristalline PH 101.

4. Composition pour capsule pharmaceutique selon la revendication 3, dans laquelle la cellulose microcristalline PH 101 est présente dans une quantité de 35,0 à 75,0 % en poids de la composition totale.

5. Composition pour capsule pharmaceutique selon la revendication 3, dans laquelle la cellulose microcristalline PH 101 a une taille de particule (d₅₀) entre 50,0 µm et 95,0 µm.

6. Composition pour capsule pharmaceutique selon la revendication 1, dans laquelle au moins un excipient pharmaceutiquement acceptable est choisi parmi un diluant, un lubrifiant ou les mélanges de ceux-ci.

7. Composition pour capsule pharmaceutique selon la revendication 6, dans laquelle le diluant est l'hydrogéno phosphate de calcium dihydraté.

8. Composition pour capsule pharmaceutique selon la revendication 6, dans laquelle le lubrifiant est le fumarate de stéaryle et de sodium.

9. Composition pour capsule pharmaceutique selon l'une quelconque des revendications précédentes, comprenant :
a. du chlorhydrate de fingolimod ;
b. de la cellulose microcristalline PH 101 ayant une taille de particule (d50) entre 50,0 µm et 95,0 µm ;
c. de l'hydrogéno phosphate de calcium dihydraté ;
d. du polyéthylène glycol 6000 P ;
e. du fumarate de stéaryle et de sodium.
